# EUROPEAN PATENT SPECIFICATION

(11) **EP 1 951 645 B1**
(45) Date of publication and mention of the grant of the patent: **16.09.2015**
(21) Application number: 06819610.4
(22) Date of filing: 20.11.2006
(51) Int. Cl.: B01J 31/14, B01J 31/18, C07F 9/46, C07C 2/36

(54) **CATALYTIC PROCESS FOR THE OLIGOMERIZATION OF OLEFINIC MONOMERS**
KATALYTISCHES VERFAHREN ZUR OLIGOMERISIERUNG VON OLEFINISCHEN MONOMEREN
PROCEDE CATALYTIQUE PERMETTANT L'OLIGOMERISATION DE MONOMERES OLEFINIQUES

(30) Priority: 21.11.2005 EP 05257159
(43) Date of publication of application: 06.08.2008
(73) Proprietor: Shell Internationale Research Maatschappij B.V., 2596 HR Den Haag (NL)
(72) Inventor: DE BOER, Eric Johannes Maria, NL-1031 CM Amsterdam (NL); VAN DER HEIJDEN, Harry, NL-1031 CM Amsterdam (NL); ON, Quoc An, NL-1031 CM Amsterdam (NL); SMIT, Johan Paul, NL-1031 CM Amsterdam (NL); VAN ZON, Arie, NL-1031 CM Amsterdam (NL)
(74) Representative: Matthezing, Robert Maarten
(86) International application number: PCT/EP2006/068659
(87) International publication number: WO 2007/057458

(56) References cited:
- WO-A-02/04119
- CARTER A ET AL: "High activity ethylene trimerisation catalysts based on diphosphine ligands" CHEMICAL COMMUNICATIONS - CHEMCOM, ROYAL SOCIETY OF CHEMISTRY, GB, vol. 2002, no. 8, 20 March 2002 (2002-03-20), pages 858-859, XP002277009 ISSN: 1359-7345 cited in the application

## Description

### Field of the Invention

The present invention relates to a process for the oligomerization of olefinic monomers.

### Background of the Invention

The efficient catalytic trimerization or tetramerization of olefinic monomers, such as the trimerization and tetramerization of ethylene to 1-hexene and 1-octene, is an area of great interest for the production of olefinic trimers and tetramers of varying degrees of commercial value. In particular, 1-hexene is a valuable comonomer for linear low-density polyethylene (LLDPE) and 1-octene is valuable as a chemical intermediate in the production of plasticizer alcohols, fatty acids, detergent alcohol and lubrication oil additives as well as a valuable comonomer in the production of polymers such as polyethylene. 1-Hexene and 1-octene can be produced by a conventional transition metal oligomerization process, although the trimerization and tetramerization routes are preferred.

Several different catalytic systems have been disclosed in the art for the trimerization of ethylene to 1-hexene. A number of these catalysts are based on chromium.

US-A-5198563 (Phillips) discloses chromium-based catalysts containing monodentate amine ligands useful for trimerizing olefins.

US-A-5968866 (Phillips) discloses an ethylene oligomerization/trimerization process which uses a catalyst comprising a chromium complex which contains a coordinating asymmetric tridentate phosphane, arsane or stibane ligand and an aluminoxane to produce alphaolefins which are enriched in 1-hexene.

US5523507 (Phillips) discloses a catalyst based on a chromium source, a 2,5-dimethylpyrrole ligand and an alkyl aluminium activator for use in the trimerization of ethylene to 1-hexene.

Chem. Commun., 2002, 8, 858-859 (BP), discloses chromium complexes of ligands of the type Ar₂PN(Me)PAr₂ (Ar = ortho-methoxy-substituted aryl group) as catalysts for the trimerization of ethylene.

WO 02/04119 (BP) discloses a catalyst for the trimerization of olefins comprising a source of chromium, molybdenum or tungsten, a ligand containing at least one phosphorus, arsenic or antimony atom bound to at least one hydrocarbyl or heterohydrocarbyl group having a polar substituent, but excluding the case where all such polar substituents are phosphane, arsane or stibane groups, and optionally an activator. The ligand used in most of the examples is (2-methoxyphenyl)₂PN(Me)P(2-methoxyphenyl)₂.

Although the catalysts disclosed in the BP documents mentioned above have good selectivity for 1-hexene within the C₆ fraction, a relatively high level of by-product formation (e.g. C₁₀ by-products) is typically observed.

WO 2005/039758 (Shell) discloses a trimerization catalyst composition and a process for the trimerization of olefinic monomers using said catalyst composition.

Catalytic systems for the tetramerization of ethylene to 1-octene have recently been described. A number of these catalysts are based on chromium.

WO 2004/056478 and WO 2004/056479 (Sasol) disclose catalyst compositions and processes for the tetramerization of olefins. The catalyst compositions disclosed in WO 2004/056478 comprise a transition metal and a heteroatomic ligand having the general formula (R)ₙA-B-C(R)ₘ where A and C are independently selected from a group which comprises phosphorus, arsenic, antimony, oxygen, bismuth, sulphur, selenium, and nitrogen, and B is a linking group between A and C, and R is independently selected from any homo or heterohydrocarbyl group of which at least one R group is substituted with a polar substituent and n and m are determined by the respective valence and oxidation state of A and/or C. The catalyst compositions disclosed in WO 2004/056479 comprise a transition metal and a heteroatomic ligand having the general formula (R')ₙA-B-C(R')ₘ where A, B, C, n and m are as defined above, and R' is independently selected from any homo or heterohydrocarbyl group.

Example 16 of WO 2004/056478 discloses an ethylene tetramerization reaction using Cr(III)acetylacetonoate, (phenyl)₂PN(isopropyl)P(2-methoxyphenyl)₂ in a ratio of 1:2 mol/mol, and MAO, with an Al:Cr atomic ratio of 136:1, at 45 °C and 45 barg. However, the reaction produced a product composition with over 24 wt% of the products having greater than 11 carbon atoms, based on the weight of all products (9.00 wt% C₁₁+ liquids and 15.11 wt% solids).

WO 2004/056480 (Sasol) discloses the tandem tetramerization and polymerisation of ethylene. Specifically, WO 2004/056480 discloses a process for polymerising olefins to produce branched polyolefins in the presence of a distinct polymerization catalyst and a distinct tetramerization catalyst, wherein the tetramerization catalyst produces 1-octene in a selectivity greater than 30% and the 1-octene produced is at least partially incorporated into the polyolefin chain.

Although the tetramerization catalysts disclosed in the Sasol documents mentioned above have good selectivity for 1-octene within the C₈ fraction, again, a relatively high level of by-product formation is observed. Typically, the by-product consists of C₆ compositions; however, only about 70 to 80 %wt. of the C₆ by-product composition is 1-hexene, with the remaining C₆ by-product comprising compounds such as methylcyclopentane and methylenecyclopentane. The presence of these remaining C₆ by-product compositions, which have very little commercial use or value, is highly undesirable from both an economic point of view as well as from a product separation point of view.

It has now been surprisingly found that the process of the present invention provides an efficient route for the trimerization and tetramerization of olefinic monomers, in particular the selective production of 1-hexene and 1-octene from ethylene while reducing the level of by-product formation, especially C₁₀ by-products, solids (i.e. heavy waxes and/or polyethylene) and C₆ compositions/isomers other than 1-hexene.

### Summary of the Invention

The present invention relates to a process for the simultaneous trimerization and tetramerization of olefinic monomers, wherein the process comprises contacting at least one olefinic monomer with a catalyst system comprising:
a) a source of chromium;
b) a ligand having the general formula (I);

   (R¹)₂P-X-P(R¹)ₘ(R²)ₙ (I)

   wherein:
   X is a bridging group of the formula -N(R³)-, wherein R³ is selected from hydrogen, a hydrocarbyl group, a substituted hydrocarbyl group, a silyl group or derivative thereof;
   the R¹ groups are independently selected from an optionally substituted aromatic group bearing a polar substituent on at least one of the ortho-positions; and
   the R² groups are independently selected from substituted or unsubstituted aromatic groups, including heteroaromatic groups, which do not contain a polar substituent at any of the ortho-positions;
   with the proviso that m is 0 or 1, n is 1 or 2 and the total of m + n is 2;
   optionally, any of the R¹ and R² groups may independently be linked to one or more of each other or to the bridging group X to form a cyclic structure; and
c) a cocatalyst,
at a pressure in the range of from below atmospheric to about 40 barg and at a temperature in the range of from about 0 °C to about 120 °C.

The present invention also relates to a process for the simultaneous trimerization and tetramerization of ethylene to 1-hexene and 1-octene with said catalyst system comprising (a), (b) and (c), and at the pressure and temperature defined above.

### Detailed Description of the Invention

As used herein, the term "trimerization" means the catalytic trimerization of an olefinic monomer to give a product composition enriched in the compound derived from the reaction of three of said olefinic monomers. The term trimerization includes the cases wherein all the olefinic monomers in the feed stream are identical as well as the cases wherein the feed stream contains two or more different olefinic monomers.

In particularly, the term "trimerization" when used in relation to the trimerization of ethylene means the trimerization of ethylene to form a C₆ alkene, especially 1-hexene.

The term "trimerization selectivity" when used in relation to the trimerization of ethylene means the amount of C₆ fraction formed within the product composition.

The term "1-hexene selectivity" when used in relation to the trimerization of ethylene means the amount of 1-hexene formed within the C₆ fraction of the product composition. The overall yield of 1-hexene in the trimerization of ethylene is the product of the "trimerization selectivity" multiplied by the "1-hexene selectivity".

The term "tetramerization" means the catalytic tetramerization of an olefinic monomer to give a product composition enriched in the compound derived from the reaction of four of said olefinic monomers. The term tetramerization includes the cases wherein all the olefinic monomers in the feed stream are identical as well as the cases wherein the feed stream contains two or more different olefinic monomers.

In particularly, the term "tetramerization" when used in relation to the tetramerization of ethylene means the tetramerization of ethylene to form a C₈ alkene, especially 1-octene.

The term "tetramerization selectivity" when used in relation to the tetramerization of ethylene means the amount of C₈ fraction formed within the product composition.

The term "1-octene selectivity" when used in relation to the tetramerization of ethylene means the amount of 1-octene formed within the C₈ fraction of the product composition. The overall yield of 1-octene in the tetramerization of ethylene is the product of the "tetramerization selectivity" multiplied by the "1-octene selectivity".

The source of chromium, component (a), for the catalyst system of the process of the present invention can include simple inorganic and organic salts of chromium. Examples of simple inorganic and organic salts are halides, acetylacetonates, carboxylates, oxides, nitrates, sulfates and the like. Further sources of chromium can also include co-ordination and organometallic complexes, for example chromium trichloride tris-tetrahydrofuran complex, (benzene)tricarbonylchromium, chromium hexacarbonyl, and the like. Preferably, the source of chromium, component (a), for the catalyst system is selected from simple inorganic and organic salts of chromium, molybdenum or tungsten.

In one embodiment of the present invention, the source of chromium, component (a), for the catalyst system is a simple inorganic or organic salt of chromium, which is soluble in a solvent such as those disclosed in WO 02/04119.

The source of chromium can also include a mixture of any combination of simple inorganic salts, simple organic salts, co-ordination complexes and organometallic complexes.

In a preferred embodiment herein, component (a) is a source of chromium (III).

Preferred sources of chromium for use herein are simple inorganic and organic salts of chromium and co-ordination or organometallic complexes of chromium. More preferred sources of chromium for use herein are the simple inorganic and organic salts of chromium, such as salts of carboxylic acids, preferably salts of alkanoic acids containing 1 to 30 carbon atoms, salts of aliphatic-β-diketones and salts of β-ketoesters (e.g. chromium (III) 2-ethylhexanoate, chromium (III) octanoate and chromium (III) acetylacetonate), and halide salts of chromium, such as chromium trichloride, chromium trichloride tris-tetrahydrofuran complex, chromium tribromide, chromium trifluoride, and chromium triiodide. Specific examples of preferred sources of chromium for use herein are chromium (III) acetylacetonate, also called chromium tris(2,4-pentanedionate), Cr(acac)₃, chromium trichloride, CrCl₃, and chromium trichloride tris-tetrahydrofuran complex, CrCl₃(THF)₃.

The ligand of the catalyst system of the process of the present invention, component (b), is of the general formula (I);

(R¹)₂P-X-P(R¹)ₘ(R²)ₙ (I)

wherein:
X is a bridging group of the formula -N(R³)-, wherein R³ is selected from hydrogen, a hydrocarbyl group, a substituted hydrocarbyl group, a silyl group or derivative thereof;
the R¹ groups are independently selected from an optionally substituted aromatic group bearing a polar substituent on at least one of the ortho-positions; and
the R² groups are independently selected from substituted or unsubstituted aromatic groups, including heteroaromatic groups, which do not contain a polar substituent at any of the ortho-positions;
with the proviso that m is 0 or 1, n is 1 or 2 and the total of m + n is 2; and
optionally, any of the R¹ and R² groups may independently be linked to one or more of each other or to the bridging group X to form a cyclic structure.

The bridging group X is of the formula -N(R³)-, wherein R³ is preferably a hydrocarbyl group, a substituted hydrocarbyl group, a silyl group or derivative thereof. Typically, R³ is selected from hydrogen or the groups consisting of alkyl, substituted alkyl, aryl, substituted aryl, aryloxy, substituted aryloxy, alkenyl, substituted alkenyl, cycloalkyl, substituted cycloalkyl, silyl groups or derivatives thereof, and alkyl or aryl groups substituted with any of these substituents or alkoxycarbonyl, carbonyloxy, alkoxy, aminocarbonyl, carbonylamino or dialkylamino groups or halogen or a nitro group. More preferably R³ is an alkyl, substituted alkyl (including heterocyclic substituted alkyl with at least one heteroatom, such as N or O, and alkyl groups substituted with a heteroatom or heteroatomic group), cycloalkyl, substituted cycloalkyl, substituted cyclic aryl, substituted aryl, aryloxy or substituted aryloxy group. Examples of suitable R³ groups include C₁-C₁₅ alkyl groups, substituted C₁-C₁₅ alkyl groups, C₂-C₁₅ alkenyl groups, substituted C₂-C₁₅ alkenyl groups, C₃-C₁₅ cycloalkyl groups, substituted C₃-C₁₅ cycloalkyl groups, C₅-C₁₅ aromatic groups, substituted C₅-C₁₅ aromatic groups, C₁-C₁₅ alkoxy groups and substituted C₁-C₁₅ alkoxy groups. Most preferred R³ groups are the C₁-C₁₅ alkyl groups, which include both linear and branched alkyl groups; suitable examples include methyl, ethyl, propyl, isopropyl, butyl, isobutyl, t-butyl, pentyl, alkyl branched pentyl groups, hexyl, alkyl branched hexyl groups, heptyl, alkyl branched heptyl groups, octyl and alkyl branched octyl groups.

Examples of suitable bridging groups include-N(methyl)-, -N(ethyl)-, -N(propyl)-, -N(isopropyl)-, - N(butyl)-, -N(*t*-butyl)-, -N(pentyl)-, -N(hexyl)-, -N(2-ethylhexyl)-, -N(cyclohexyl)-, -N(1-cyclohexylethyl)-, - N(2-methylcyclohexyl)-, -N(benzyl)-, -N(phenyl)-, -N(2-octyl)-, -N(p-methoxyphenyl)-, -N(p-t-butylphenyl)-,-N((CH₂)₃-N-morpholine)-, -N(Si(CH₃)₃)-,-N(CH₂CH₂CH₂Si(OMe)₃))-, -N(decyl)- and -N(allyl)-.

The term "hydrocarbyl" as used herein refers to a group only containing carbon and hydrogen atoms. The hydrocarbyl group may be a saturated or unsaturated, linear or branched alkyl, a non-aromatic ring or an aromatic ring. Unless otherwise stated, the preferred hydrocarbyl groups for use herein are those containing from 1 to 20 carbon atoms.

The term "substituted hydrocarbyl" as used herein refers to hydrocarbyl groups which contain one or more inert heteroatom containing functional groups. By "inert heteroatom containing functional groups" is meant that the functional groups do not interfere to any substantial degree with the trimerization and tetramerization process.

The term "aromatic" as used herein, refers to a monocyclic or polycyclic, aromatic or heteroaromatic ring having from 5 to 14 ring atoms, optionally containing from 1 to 3 heteroatoms selected from N, O and S. Preferably, the aromatic groups are monocyclic or polycyclic aromatic rings, such as cyclopentadienyl (which can also include ferrocenyl groups), phenyl, naphthyl or anthracenyl. Unless otherwise stated, the preferred aromatic groups are monocyclic or polycyclic aromatic rings having from 5 to 10 ring atoms, more preferred aromatic groups are monocyclic aromatic rings containing from 5 to 6 carbon atoms, such as phenyl and cyclopentadienyl, and a most preferred aromatic group is a.phenyl group. The term "substituted aromatic" as used herein means that the aromatic group may be substituted with one or more substituents.

By the term "ortho-position" when used in relation to substituents on aromatic R¹ and/or R² groups, it is meant that the substituent is in the ortho position relative to the atom bonded to the phosphorus atom.

The substituents on the R¹ and/or R² groups can contain carbon atoms and/or heteroatoms. The substituents may be either polar or non-polar. Suitable substituents include hydrocarbyl groups which may be straight-chain or branched, saturated or unsaturated, aromatic or non-aromatic. The hydrocarbyl substituents may optionally contain heteroatoms such as Si, S, N or O. Suitable aromatic hydrocarbyl substituents include monocyclic and polycyclic aromatic groups, preferably having from 5 to 10 carbon atoms in the ring, such as phenyl and C₁-C₄ alkyl phenyl groups. Suitable non-aromatic hydrocarbyl substituents include linear or branched alkyl or cycloalkyl groups, preferably having from 1 to 10 carbon atoms, more preferably 1 to 4 carbon atoms.

Other suitable substituents on the R¹ and/or R² groups include halides such as chloride, bromide and iodide, thiol, -OH, A¹-O-, -S-A¹, -CO-A¹, -NH₂, -NHA¹,-NA¹A², -CO-NA¹A², -NO₂, =O, in which A¹ and A², independently, are non-aromatic groups preferably having from 1 to 10 carbon atoms, more preferably 1 to 4 carbon atoms, e.g. methyl, ethyl, propyl and isopropyl.

When the R¹ and/or R² groups of the ligand are substituted, preferred substituents are hydrocarbyl groups. Particularly preferred hydrocarbyl substituents are C₁-C₄ alkyl groups, preferably methyl, ethyl, propyl, isopropyl, butyl, isobutyl, most preferably methyl.

In one embodiment of the ligand of the catalyst system of the process of the present invention, component (b), m is 1 and n is 1. In another embodiment of the ligand of the catalyst system of the process of the present invention, component (b), m is 0 and n is 2. Typically, in the ligand of the catalyst system of the process of the present invention, component (b), m is 0 and n is 2.

The R¹ groups of the ligand of the catalyst system of the process of the present invention, component (b), are independently selected from optionally substituted aromatic groups, each bearing a polar substituent on at least one of the ortho-positions. For the avoidance of doubt, the phrase "bearing a polar substituent on at least one of the ortho-positions" means that, in the same ligand, the R¹ group is substituted with a polar substituent on one or both of its ortho positions.

The term "optionally substituted" in relation to the R¹ groups of the ligand of the catalyst system of the process of the present invention, component (b), which are independently selected from optionally substituted aromatic groups, each bearing a polar substituent on at least one of the ortho-positions, means that, in addition to the polar substituent on at least one of the ortho-positions, the same R¹ group may contain one or more other substituents.

Polar is defined by IUPAC as an entity with a permanent electric dipole moment. Therefore, as used herein, the term "polar substituents" means a substituent which incorporates a permanent electric dipole moment.

Suitable polar substituents for use herein include but are not necessarily limited to, optionally branched C₁-C₂₀ alkoxy groups, i.e. the R¹ and/or R² groups are substituted with a hydrocarbyl group connected through an oxygen bridging atom; optionally substituted C₅-C₁₄ aryloxy groups, i.e. the R¹ and/or R² groups are substituted with an optionally substituted aromatic group connected through an oxygen bridging atom; optionally branched C₁-C₂₀ alkoxy(C₁-C₂₀)alkyl groups, i.e. the R¹ and/or R² groups are substituted with a C₁-C₂₀ hydrocarbyl group bearing a C₁-C₂₀ alkoxy group; hydroxyl; amino; (di-)C₁-C₆ alkylamino; nitro; C₁-C₆ alkylsulphonyl; C₁-C₆ alkylthio(C₁-C₆)alkyl groups; sulphate; heterocyclic groups, especially with at least one N and/or O ring atom; and tosyl groups.

Examples of suitable polar substituents include methoxy, ethoxy, isopropoxy, phenoxy, decyloxy, dodecyloxy, tetradecyloxy, hexadecyloxy, octadecyloxy, eicosanoxy, pentafluorophenoxy, trimethylsiloxy, dimethylamino, methylsulphonyl, tosyl, methoxymethyl, methylthiomethyl, 1,3-oxazolyl, hydroxyl, amino, methoxymethyl, phosphino, arsino, stibino, sulphate, nitro and the like.

Preferably, the polar substituents in the R¹ groups are independently selected from optionally branched C₁-C₂₀ alkoxy groups, optionally substituted C₅-C₁₄ aryloxy groups, and optionally branched C₁-C₂₀ alkyl(C₁-C₂₀)alkoxy groups. More preferably, the polar substituents are independently selected from optionally branched C₁-C₂₀ alkoxy groups, especially optionally branched C₁-C₆ alkoxy groups such as, for example, methoxy, ethoxy or isopropoxy of which methoxy is a particularly preferred polar substituent; alternatively, longer optionally branched C₁-C₂₀ alkoxy groups such as optionally branched C₈-C₂₀ alkoxy groups, for example decyloxy, dodecyloxy, tetradecyloxy, hexadecyloxy, octadecyloxy or eicosanoxy groups, of which eicosanoxy is preferred, may be preferred as the polar substituents in order to increase the solubility of the ligand in organic media.

In one embodiment, the R¹ group is independently selected from substituted or unsubstituted aromatic groups bearing an optionally branched C₁-C₂₀ alkoxy group on at least one of the ortho-positions, such as an o-anisyl group.

It is preferred that the R¹ groups of the ligand of the catalyst system of the process of the present invention, component (b), are the same and bear the same number and type of polar substituent(s). It is particularly preferred that each of said R¹ groups bears a polar substituent on only one of the two available ortho-positions.

The R² groups of the ligand of the catalyst system of the process of the present invention, component (b), are independently selected from substituted or unsubstituted aromatic groups, including substituted or unsubstituted heteroaromatic groups, which do not contain a polar substituent at any of the ortho-positions.

In one embodiment of the ligand of the catalyst system of the process of the present invention, component (b), the R² groups may be independently selected from a group comprising optionally substituted benzyl, phenyl, tolyl, xylyl, mesityl, biphenyl, naphthyl, anthracenyl, phenoxy, tolyloxy, thiophenyl, pyridyl, thiophenoxy, ferrocenyl and tetrahydrofuranyl groups. In another embodiment of the ligand, said R² groups may be independently selected from a group comprising optionally substituted phenyl, tolyl, biphenyl, naphthyl and thiophenyl groups.

In a further embodiment of the ligand of the catalyst system of the process of the present invention, said R² groups are independently selected from optionally substituted phenyl groups which do not contain a polar substituent at any of the ortho-positions, or alternatively, do not contain any polar substituents at all. Any polar substituent present in said R² groups may be electron donating. Said R² groups may optionally contain non-polar substituent.

IUPAC defines non-polar as an entity without a permanent electric dipole moment.

Suitable non-polar substituents may be a methyl, ethyl, propyl, butyl, isopropyl, isobutyl, tert-butyl, pentyl, hexyl, cyclopentyl, 2-methylcyclohexyl, cyclohexyl, cylopentadienyl, phenyl, bi-phenyl, naphthyl, tolyl, xylyl, mesityl, ethenyl, propenyl and benzyl group, or the like. Preferably, the non-polar substituent is not electron donating.

In one specific embodiment of the ligand of the catalyst system of the process of the present invention, component (b), said R² group is an unsubstituted phenyl group.

Optionally, any of the R¹ and R² groups may independently be linked to one or more of each other or to the bridging group X to form a cyclic structure. In particular, when n is 2 then the two R² groups may optionally be linked together to form a cyclic structure incorporating the phosphorus atom.

In another embodiment of the present invention, one or both of the phosphorus atoms of the ligand of the catalyst system of the process of the present invention may be independently oxidised by S, Se, N or O. Typically, neither of the phosphorus atoms of the second ligand are oxidised by S, Se, N or O.

In a further embodiment of the present invention, the ligand of the catalyst system of the process of the present invention may also optionally contain multiple (R¹)₂P-X-P(R¹)ₘ(R²)ₙ units. Non-limiting examples of such ligands include ligands where the individual units are coupled either via one or more of the R¹ or R² groups or via the bridging group X. Typically, the ligand does not contain multiple (R¹)₂P-X-P(R¹)ₘ(R²)ₙ units. The ligands according to formula (I) can be prepared using procedures known to one skilled in the art or disclosed in published literature. Examples of such compounds include: (2-methoxyphenyl)₂PN(methyl)P(2-methoxyphenyl)(phenyl), (2-methoxyphenyl)₂PN(methyl)P(phenyl)₂, (2-ethoxyphenyl)₂PN(methyl)P(2-ethoxyphenyl)(phenyl), (2-ethoxyphenyl)₂PN(methyl)P(phenyl)₂, (2-methoxyphenyl)(2-ethoxyphenyl)PN(methyl)P(2-methoxyphenyl)(phenyl), (2-methoxyphenyl)(2-ethoxyphenyl)PN(methyl)P(phenyl)₂, (2-isopropoxyphenyl)₂PN(methyl)P(2-isopropoxyphenyl)(phenyl), (2-isopropoxyphenyl)₂PN(methyl)P(phenyl)₂, (2-methoxyphenyl)₂PN(methyl)P(2-methoxyphenyl)(3-methoxyphenyl), (2-methoxyphenyl)₂PN(methyl)P(3-methoxyphenyl)₂, (2-methoxyphenyl)₂PN(methyl)P(2-methoxyphenyl)(4-methoxyphenyl), (2-methoxyphenyl)₂PN(methyl)P(4-methoxyphenyl)₂, (2-methoxyphenyl)₂PN(methyl)P(2-methoxyphenyl)(4-fluorophenyl), (2-methoxyphenyl)₂PN(methyl)P(4-fluorophenyl)₂, (2-methoxyphenyl)₂PN(methyl)P(2-ethoxyphenyl)(4-fluorophenyl), (2-methoxyphenyl)₂PN(methyl)P(2-methoxyphenyl)(4-dimethylamino-phenyl) (2-methoxyphenyl)₂PN(methyl)P(4-dimethylamino-phenyl)₂, (2-methoxyphenyl)₂PN(methyl)P(2-methoxyphenyl)(4-(4-methoxyphenyl)-phenyl), (2-methoxyphenyl)₂PN(methyl)P(4-(4-methoxyphenyl)-phenyl)₂, (2-methoxyphenyl)₂PN(methyl)P(2-methoxyphenyl)(4-dimethylamino-phenyl), (2-methoxyphenyl)₂PN(methyl)P(4-dimethylamino-phenyl)₂, (2-methoxyphenyl)₂PN(methyl)P(2-methoxyphenyl)(4-(4-methoxyphenyl)-phenyl), (2-methoxyphenyl)₂PN(methyl)P(4-(4-methoxyphenyl)-phenyl)₂, (2-methoxyphenyl)₂PN(methyl)P(2-methoxyphenyl)(o-ethylphenyl), (2-methoxyphenyl)₂PN(methyl)P(o-ethylphenyl)₂, (2-methoxyphenyl)₂PN(methyl)P(2-methoxyphenyl)(2-naphthyl), (2-methoxyphenyl)₂PN(methyl)P(2-naphthyl)₂, (2-methoxyphenyl)₂PN(methyl)P(2-methoxyphenyl)(p-biphenyl), (2-methoxyphenyl)₂PN(methyl)P(p-biphenyl)₂, (2-methoxyphenyl)₂PN(methyl)P(2-methoxyphenyl)(p-methylphenyl), (2-methoxyphenyl)₂PN(methyl)P(p-methylphenyl)₂, (2-methoxyphenyl)₂PN(methyl)P(2-methoxyphenyl)(2-thiophenyl), (2-methoxyphenyl)₂PN(methyl)P(2-thiophenyl)₂, (2-methoxyphenyl)₂PN(methyl)P(2-methoxyphenyl)(m-methylphenyl), (2-methoxyphenyl)₂PN(methyl)P(m-methylphenyl)₂, (2-methoxyphenyl)₂PN(ethyl)P(2-methoxyphenyl)(phenyl), (2-methoxyphenyl)₂PN(ethyl)P(phenyl)₂, (2-methoxyphenyl)₂ PN(propyl)P (2-methoxyphenyl)(phenyl), (2-methoxyphenyl)₂ PN(propyl)P (phenyl)₂, (2-methoxyphenyl)₂PN(isopropyl)P(2-methoxyphenyl)(phenyl), (2-methoxyphenyl)₂PN(isopropyl)P(phenyl)₂, (2-methoxyphenyl)₂PN(butyl)P(2-methoxyphenyl)(phenyl), (2-methoxyphenyl)₂PN(butyl)P(phenyl)₂, (2-methoxyphenyl)₂PN(t-butyl)P(2-methoxyphenyl)(phenyl), (2-methoxyphenyl)₂PN(t-butyl)P(phenyl)₂, (2-methoxyphenyl)₂PN(phenyl)P(2-methoxyphenyl)(phenyl), (2-methoxyphenyl)₂PN(phenyl)P(phenyl)₂, methoxyphenyl)₂PN(cyclohexyl)P(2-methoxyphenyl)(phenyl), (2-methoxyphenyl)₂PN(cyclohexyl)P(phenyl)₂, (2-methoxyphenyl)₂PN(1-cyclohexylethyl)P(2-methoxyphenyl)(phenyl), (2-methoxyphenyl)₂PN(1-cyclohexylethyl)P(phenyl)₂, (2-methoxyphenyl)₂PN(2-methylcyclohexyl)P(2-methoxyphenyl)(phenyl), (2-methoxyphenyl)₂PN(2-methylcyclohexyl)P(phenyl)₂, (2-methoxyphenyl)₂PN(decyl)P(2-methoxyphenyl)(phenyl), (2-methoxyphenyl)₂PN(decyl)P(phenyl)₂, (2-methoxyphenyl)₂PN(allyl)P(2-methoxyphenyl)(phenyl), (2-methoxyphenyl)₂PN(allyl)P(phenyl)₂, (2-methoxyphenyl)₂PN(p-methoxyphenyl)P(2-methoxyphenyl)(phenyl), (2-methoxyphenyl)₂PN(p-methoxyphenyl)P(phenyl)₂, (2-methoxyphenyl)₂PN(p-*t-*butylphenyl)P(2-methoxyphenyl)(phenyl), (2-methoxyphenyl)₂PN(p-*t*-butylphenyl)P(phenyl)₂, (2-methoxyphenyl)₂PN((CH₂)₃-N-morpholine)P(2-methoxyphenyl)(phenyl), (2-methoxyphenyl)₂PN((CH₂)₃-N-morpholine)P(phenyl)₂, (2-methoxyphenyl)₂PN(Si(CH₃)₃)P(2-methoxyphenyl)(phenyl), (2-methoxyphenyl)₂PN(Si(CH₃)₃)P(phenyl)₂, (2-methoxyphenyl)₂P(=Se)N(isopropyl)P(2-methoxyphenyl) (phenyl) , (2-methoxyphenyl)₂P(=Se)N(isopropyl)P(phenyl)₂, (2-methoxyphenyl)₂PN(benzyl)P(2-methoxyphenyl)(phenyl), (2-methoxyphenyl)₂PN(benzyl)P(phenyl)₂, (2-methoxyphenyl)₂PN(1-cyclohexyl-ethyl)P(2-methoxyphenyl)(phenyl), (2-methoxyphenyl)₂PN(1-cyclohexyl-ethyl)P(phenyl)₂, (2-methoxyphenyl)₂PN[CH₂CH₂CH₂Si(OMe₃)]P(2-methoxyphenyl)(phenyl), (2-methoxyphenyl)₂PN[CH₂CH₂CH₂Si(OMe₃)]P(phenyl)₂, (2-methoxyphenyl)₂PN(2-methylcyclohexyl)P(2-methoxyphenyl)(phenyl), (2-methoxyphenyl)₂PN(2-methylcyclohexyl)P(phenyl)₂ (2-methoxyphenyl)₂P(=S)N(isopropyl)P(2-methoxyphenyl)(phenyl), (2-methoxyphenyl)₂P(=S)N(isopropyl)P(phenyl)₂, (2-eicosanoxyphenyl)₂PN(methyl)P(2-eicosanoxyphenyl)(phenyl), (2-eicosanoxyphenyl)₂PN(methyl)P(phenyl)₂, (2-methoxyphenyl) (2-eicosanoxyphenyl)PN(methyl)P(phenyl)₂, (2-methoxyphenyl)(2-eicosanoxyphenyl)PN(methyl)P(2-eicosanoxyphenyl)(phenyl), (2-eicosanoxyphenyl)₂PN(methyl)P(4-eicosanoxyphenyl)(phenyl), (2-methoxyphenyl)(2-eicosanoxyphenyl)PN(methyl)P(4-eicosanoxyphenyl)(phenyl), (2-eicosanoxyphenyl)₂PN(methyl)P(4-eicosanoxyphenyl)₂, (2-methoxyphenyl)(2-eicosanoxyphenyl)PN(methyl)P(4-eicosanoxyphenyl)₂, (2-eicosanoxyphenyl)₂PN(methyl)P(2-eicosanoxyphenyl)(4-eicosanoxyphenyl), (2-methoxyphenyl)( 2-eicosanoxyphenyl)PN(methyl)P(2-eicosanoxyphenyl)(4-eicosanoxyphenyl), and the like.

The cocatalyst, component (c), may in principle be any compound or mixture of compounds that generates an active catalyst system with the source of chromium, component (a), and the ligand, component (b).

Compounds which are suitable for use as a cocatalyst include organoaluminium compounds, organoboron compounds, organic salts, such as methyllithium and methylmagnesium bromide and inorganic acids and salts, such as tetrafluoroboric acid etherate, silver tetrafluoroborate, sodium hexafluoroantimonate and the like.

Particularly preferred cocatalysts are organoaluminium compounds. Suitable organoaluminium compounds for use herein are those having the formula AlR⁴₃, wherein each R⁴ group is independently selected from C₁-C₃₀ alkyl (preferably C₁-C₁₂ alkyl), oxygen containing moieties or halides, and compounds such as LiAlH₄ and the like. Non-limiting examples of suitable organoaluminium compounds include trimethylaluminium (TMA), triethylaluminium (TEA), tri-n-butyl aluminium, tri-isobutylaluminium (TIBA), tri-n-octylaluminium, methylaluminium dichloride, ethylaluminium dichloride, dimethylaluminium chloride, diethylaluminium chloride and aluminoxanes (also called alumoxanes). Mixtures of organoaluminium compounds are also suitable for use herein.

In a preferred embodiment herein, the cocatalyst is an aluminoxane cocatalyst. These aluminoxane cocatalysts may comprise any aluminoxane compound or a mixture of aluminoxane compounds. Aluminoxanes may be prepared by the controlled addition of water to an alkylaluminium compound, such as those mentioned above, or are available commercially. Non-limiting examples of suitable aluminoxanes include methyl aluminoxane (MAO), modified methyl aluminoxane (MMAO), tetraisobutyl dialuminoxane (TIBAO), tetra-n-butyl dialuminoxane and tetra-n-octyl dialuminoxane. In this context it should be noted that the term "aluminoxane" as used within this specification includes commercially available aluminoxanes, which are derived from the corresponding trialkylaluminium by addition of water and which may contain from 2 to 15 %wt., typically about 5 %wt., but optionally about 10 %wt., of aluminium.

Other suitable co-catalysts include those mentioned in WO 02/04119, WO 2004/056478 and WO 2004/056479, the disclosures of which are incorporated herein in their entirety by reference.

The quantity of cocatalyst in the catalyst system the present invention is typically enough to provide a ratio in the range from 0.1 to 20,000, preferably from 1 to 2000, more preferably 1 to 1000, most preferably 1 to 500, aluminium or boron atoms per atom of chromium.

In one specific embodiment of the present invention the catalyst system of the process of the present invention comprises:
a) a source of chromium;
b) a ligand having the general formula (I);

   (R¹)₂P-X-P(R²)₂ (I)

   wherein X, R¹ and R² are as defined above; and
c) a cocatalyst.

In another specific embodiment of the present invention the catalyst system of the process of the present invention comprises:
a) a source of chromium;
b) a ligand having the general formula (I);

   (R¹)₂P-X-P(R¹)(R²) (I)

   wherein X, R¹ and R² are as defined above; and
c) a cocatalyst.

The catalyst system of the process of the present invention may independently comprise more than one ligand as defined above.

The amount of chromium, namely component (a), and the amount of ligand, component (b), can be present in the system in a molar ratio in the range of from 100:1 to 1:100, preferably from 10:1 to 1:10. More preferably, the chromium, component (a), and the ligand, component (b), are present in a molar ratio in the range of from 3:1 to 1:3. Most preferably the amount of component (a) and the amount of component (b) are present in a molar ratio of from 1:0.9 to 1:1.1.

The three catalyst components of the catalyst system, (a), (b) and (c), may be added together simultaneously or sequentially in any order so as to provide an active catalyst. The three catalyst components of the catalyst system, (a), (b) and (c), may be contacted in the presence of any suitable solvent. Suitable solvents are known to those skilled in the art, suitable solvents may include any inert solvent that does not react with the co-catalyst component, such as saturated aliphatic, unsaturated aliphatic, aromatic, halogenated hydrocarbons and ionic liquids. Typical solvents include, but are not limited to, benzene, toluene, xylene, ethylbenzene, cumene, propane, butane, pentane, heptane, decane, dodecane, tetradecane, methylcyclohexane, methylcycopentane, cyclohexane, 1-hexene, 1-octene and the like. Other examples of suitable solvents are those disclosed in WO 02/04119, such as hydrocarbon solvents and polar solvents such as diethyl ether, tetrahydrofuran, acetonitrile and the like.

In one embodiment of the present invention, the catalyst system of the process of the present invention is formed by adding the co-catalyst component, (c), to a catalyst precursor composition comprising components (a) and (b).

The catalyst system of the present invention may be prepared either in the presence (i.e. "in-situ") or absence of the olefinic monomer. The three catalyst components of the catalyst system, (a), (b) and (c), may be combined fully in the absence of the olefinic monomer, or the olefinic monomer may be included prior to contacting the components of the catalyst system, simultaneously with the components of the catalyst system or at any point in the process of contacting the components of the catalyst.

The three components of the catalyst system, (a), (b) and (c), may be combined at a temperature in the range of from -100 to 200 °C, preferably 0 to 150 °C, more preferably 20 to 100 °C.

The catalyst system of the process of the present invention may be unsupported or supported on a support material. Examples of suitable support materials can be found in WO 02/04119, WO 2004/056478 and WO 2004/056479.

The olefinic monomers suitable for use in the trimerization and tetramerization process of the present invention can be any olefinic monomers, which can be converted into a trimer or tetramer. Suitable olefinic monomers include, but are not necessarily limited to, ethylene, propylene, optionally branched C₄-C₂₄, preferably C₄-C₂₀, α-olefins, optionally branched C₄-C₂₄, preferably C₄-C₂₀, internal olefins, optionally branched C₄-C₂₄, preferably C₄-C₂₀, vinylidene olefins, optionally branched C₄-C₂₄, preferably C₄-C₂₀, cyclic olefins and optionally branched C₄-C₂₄, preferably C₄-C₂₀, dienes, as well as optionally branched C₄-C₂₄, preferably C₄-C₂₀, functionalized olefins. Examples of suitable olefinic monomers include, but are not necessarily limited to, linear α-olefins, such as ethylene, propylene, 1-butene, 1-pentene, 1-hexene, 1-heptene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene and 1-eicosene; branched α-olefins such as 4-methylpent-1-ene and 2-ethyl-1-hexene; linear and branched internal-olefins such as 2-butene; styrene; cyclohexene; norbornene and the like.

Mixtures of olefinic monomers can also be used in the process of the present invention.

Preferred olefinic monomers for use in the trimerization and tetramerization process of the present invention are propylene and ethylene. Especially preferred is ethylene.

The catalyst system and process of the present invention are particularly useful for the simultaneous trimerization and tetramerization of ethylene to 1-hexene and 1-octene.

The simultaneous trimerization and tetramerization reaction can be performed in solution phase, slurry phase, gas phase or bulk phase.

When the simultaneous trimerization and tetramerization is performed in solution or slurry phase, a diluent or solvent, which is substantially inert under trimerization and tetramerization conditions may be employed. Suitable diluents or solvents are aliphatic and aromatic hydrocarbons, halogenated hydrocarbons and olefins which are substantially inert under trimerization and tetramerization conditions may be employed, such as those disclosed in WO 02/04119, WO2004/056478 and WO2004/056479.

The trimerization and tetramerization process of the present invention may be performed in any one of a number of suitable reactors, which are well known to one skilled in the art. Typically the trimerization and tetramerization process of the present invention is carried out in a batch, semi-batch or continuous mode.

The simultaneous trimerization and tetramerization process of the present invention may be carried out under the following range of reaction conditions. Typically, the temperature will be in the range from about 0 °C to about 120 °C, preferably from about 10 °C to about 110 °C, more preferably from about 20 °C to about 100 °C, even more preferably from about 40 °C to about 100 °C. The process of present invention may also conveniently be performed at temperature range of from about 20 °C to about 70 °C. However, it may be commercially desirable to perform the process of the present invention at an elevated temperature, therefore, the process of the present invention is highly suitable to be applied at a temperature in the range of from about 70 °C to about 90 °C. The pressure range under which the process of the present invention may be performed is typically in the range of from below atmospheric pressure to about 40 barg. Preferably, the pressure will be in the range from about 0.1 to about 40 barg, more preferably from about 0.5 to about 38 barg, especially in the range of from about 1 to about 35 barg. Temperatures and pressures outside those stated above may also be employed, however, the reaction product will either have an excess of heavy and/or solid by-products or an insignificant amount of the trimer or tetramer.

By varying the temperature and pressure it is possible for the ratio of trimers and tetramers produced in the process of the present invention to be varied. The amount of trimers produced in the process of the present invention typically increases with increasing temperature. The amount of tetramers produced in the process of the present invention typically increases with increasing pressure. The amount of heavy (C₁₂₊) and/or solid by-products also appears to increase with increasing pressure in the process for the simultaneous trimerization and tetramerization of ethylene to 1-hexene and 1-octene. The amount of by-products appears to decrease with increasing temperature, although the amount of the tetramer produced also appears to decrease with increasing temperature.

Therefore, the process of the present invention can be used as a tuneable process for the trimerization and tetramerization of olefinic monomers. By the term "tuneable" as used herein, it is meant that by varying the reaction conditions of the process of the present invention, the amount of trimers and tetramers in the product composition produced by the process of the present invention may be varied. This may be useful for a tuneable, continuous or semi-continuous, process for the trimerization and tetramerization of olefinic monomers, wherein the product composition can be changed (e.g. from producing a higher proportion of trimers to a higher proportion of tetramers, or vice-versa,) by changing the reactor conditions without having to interrupt the olefinic monomer feed or the trimerization and tetramerization product flow. In particular, this may be especially useful for a tuneable, continuous or semi-continuous, process for the trimerization and tetramerization of ethylene, wherein the product composition can be changed (e.g. from producing a higher proportion of 1-hexene to a higher proportion of 1-octene, or vice-versa) by changing the reactor conditions without having to interrupt the olefinic monomer feed or the trimerization and tetramerization product flow.

In one embodiment of the present invention, there is a process for the trimerization and tetramerization of olefinic monomers, wherein the process comprises contacting at least one olefinic monomer under trimerization and tetramerization reaction conditions with a catalyst system of the process of the present invention, wherein the process is a continuous or semi-continuous process and the reaction conditions are varied during the process. Variation of the reaction conditions can be performed to make continual adjustments to a process to ensure a consistent product slate or can be performed to a process to change the product slate produced. A preferred version of this embodiment is a process for the trimerization and tetramerization of ethylene, wherein the process comprises contacting ethylene with a catalyst system of the process of the present invention, wherein the process is a continuous or semi-continuous process and the reaction conditions are varied during the process.

Separation of the products, reactant and catalyst can be performed by any technique known to one skilled in the art, such as distillation, filtration, centrifugation, liquid/liquid separation, extraction, etc.

Further details regarding reactors, solvents, separation techniques, and the like, can be found in WO 02/04119.

The use of the process of the present invention for the catalytic trimerization and tetramerization of olefinic monomers provides a simplified method of producing trimers and tetramers of the olefinic monomer with reduced formation of by-products compared with equivalent trimerization and tetramerization processes. In particular, the use of the process of the present invention for the catalytic trimerization and tetramerization of ethylene to 1-hexene and 1-octene provides a process with very high selectivity for 1-hexene and 1-octene over all the other products formed in the C₆ and C₈ fractions respectively and with reduced formation of by-products compared with equivalent trimerization and tetramerization processes.

The overall yield of 1-hexene and 1-octene in the process for the trimerization and tetramerization of ethylene of the present invention depends upon the reaction conditions employed.

Typically, the trimerization and tetramerization selectivity (i.e. the amount of trimers and tetramers of the olefinic monomers in the overall product composition) of the process of the present invention is at least 65 %wt, preferably at least 70 %wt, more preferably at least 75 %wt, of the overall product composition. The trimerization and tetramerization selectivity for the trimerization and tetramerization of ethylene (i.e. the amount of C₆ and C₈ fraction in the overall product composition) using the process of the present invention is at least 65 %wt, preferably at least 70 %wt, more preferably at least 75 %wt, of the overall product composition.

The amount of 1-hexene produced by the trimerization and tetramerization of ethylene using the process of the present invention is typically in the range of from 10 %wt to 90 %wt, preferably from 11 %wt to 85 %wt, more preferably from 12 %wt to 80 %wt, of the overall product composition. The amount of 1-octene produced by the trimerization and tetramerization of ethylene using the process of the present invention is typically in the range of from 10 %wt to 90 %wt, preferably from 11 %wt to 85 %wt, more preferably from 12 %wt to 80 %wt, of the overall product composition.

The 1-hexene selectivity (i.e. the amount of 1-hexene present in the C₆ fraction of the product composition) in the trimerization and tetramerization of ethylene using the process of the present invention is preferably at least 85 %wt, more preferably at least 90 %wt, most preferably at least 92 %wt of the C₆ fraction of the product composition.

The 1-octene selectivity (i.e. the amount of 1-octene present in the C₈ fraction of the product composition) in the trimerization and tetramerization of ethylene using the process of the present invention is preferably at least 85%wt, more preferably at least 90 %wt, most preferably at least 92 %wt of the C₈ fraction of the product composition.

The amount of solids produced in the trimerization and tetramerization of ethylene using the process of the present invention is typically at most about 5 %wt. Lower levels of solid olefin waxes and polyethylene produced in the trimerization and tetramerization of ethylene are desirable in commercial operations as this can reduce the amount of fouling of the reactor equipment, reduce the amount of waste by-products and reduce the amount of operational "downtime" due to maintenance and cleaning of the reactor equipment.

The amount of C₁₀ produced in the trimerization and tetramerization of ethylene using the process of the present invention is typically at most about 10 %wt.

In one embodiment of the present invention, the olefinic product composition of the trimerization and tetramerization of ethylene using the process of the present invention typically comprises a combined total content of 1-hexene and 1-octene of at least 70 %wt of the overall product composition, wherein the 1-hexene content is at least 10 %wt of the overall product composition, the 1-hexene selectivity is at least 90 %wt of the C₆ fraction of the product composition, the 1-octene content is at least 10 %wt of the overall product composition, the 1-octene selectivity is at least 90 %wt of the C₈ fraction of the product composition, and the amount of solids produced is at most about 5 %wt of the overall product composition.

In another embodiment of the present invention, the olefinic product composition of the trimerization and tetramerization of ethylene using the process of the present invention comprises a total content of compounds other than 1-hexene and 1-octene of at most 35 %wt of the overall product composition, preferably at most 30 %wt and more preferably at most 27 %wt, wherein the 1-hexene content is at least 10 %wt of the overall product composition, the 1-hexene selectivity is at least 90 %wt of the C₆ fraction of the product composition, the 1-octene content is at least 10 %wt of the overall product composition, the 1-octene selectivity is at least 90 %wt of the C₈ fraction of the product composition, and the amount of solids produced is at most about 5 %wt of the overall product composition.

The process of the present invention is illustrated by the following non-limiting examples.

### Examples

### General Procedures and Characterisation

All chemicals used in preparations were purchased from Aldrich and used without further purification unless mentioned otherwise.

All the operations with the catalyst systems were carried out under nitrogen atmosphere. All solvents used were dried using standard procedures. Anhydrous toluene (99.8% purity) was dried over 4Å molecular sieves (final water content of about 3 ppm). Anhydrous heptane (99.8% purity) was dried by passage over 4Å molecular sieves (final water content of about 1 ppm).

Ethylene (99.5% purity) was purified over a column containing 4Å molecular sieves and BTS catalyst (BASF) in order to reduce water and oxygen content to <1 ppm.

The oligomers obtained were characterised by Gas Chromatography (GC), in order to evaluate oligomer distribution using a HP 5890 series II apparatus and the following chromatographic conditions:
Column: HP-1 (cross-linked methyl siloxane), film thickness = 0.25µm, internal diameter = 0.25 mm, length 60 m (by Hewlett Packard); injection temperature: 325°C; detection temperature: 325°C; initial temperature: 40°C for 10 minutes; temperature programme rate: 10.0°C/minute; final temperature: 325°C for 41.5 minutes; internal standard: *n*-hexylbenzene. The yields of the C₄-C₃₀ olefins were obtained from the GC analysis.

The term "trimerization selectivity" when used in relation to the trimerization of ethylene means the amount of C₆-fraction formed within the product composition, as determined by GC.

The term "tetramerization selectivity" when used in relation to the tetramerization of ethylene means the amount of C₈-fraction formed within the product composition, as determined by GC.

The term "1-hexene selectivity" when used in relation to the trimerization of ethylene means the amount of 1-hexene formed within the C₆-fraction of the product composition, as determined by GC. The overall yield of 1-hexene in the trimerization of ethylene is the product of the "trimerization selectivity" multiplied by the "1-hexene selectivity".

The term "1-octene selectivity" when used in relation to the tetramerization of ethylene means the amount of 1-octene formed within the C₈-fraction of the product composition, as determined by GC. The overall yield of 1-octene in the tetramerization of ethylene is the product of the "tetramerization selectivity" multiplied by the "1-octene selectivity".

The amount of "solids", mainly consisting of heavy wax and polyethylene, has been determined by weighing, after its isolation from the reactor wall and appendages, followed by washing with toluene on a glass filter (P3) and by vacuum drying.

The amount of "total product" is the sum of the amount of largely olefinic product derived from GC analysis and the amount of solids.

The NMR data was obtained at room temperature with a Varian 300 MHz or 400 MHz apparatus.

### Catalyst systems

The catalyst compositions of the present invention were prepared from catalyst precursor compositions containing ligands A, B, C, and D and a chromium source, these components are described below.

### Chromium source

Chromium trichloride tris(tetrahydrofuran) complex, i.e. CrCl₃(THF)₃, and chromium tris(2,4-pentanedionate), also called chromium tris(acetylacetonate), i.e. Cr(acac)₃, have been used as the chromium sources in the simultaneous tri- and tetramerization reactions of ethylene

### Ligand component A (comparative)

The (2-methoxyphenyl)(phenyl)PN(CH₃)P(2-methoxyphenyl)(phenyl) ligand was prepared by first forming a suspension of 0.42 g lithium (60 mmol) in 80 ml of THF, to which was added 9.66 g of (2-methoxyphenyl)₂P(phenyl) (30 mmol) at 0 °C under an argon atmosphere. The mixture was stirred for 4 hours, after which time a 5 ml aliquot of methanol was added. 60 ml of toluene was added to the mixture, after which the solution was extracted with two 40 ml portions of water. The extracted toluene solution was then concentrated to a volume of approximately 20 ml, which resulted in formation of a suspension. The concentrated toluene solution was filtered, and 4.6 g of C₂Cl₆ was added to the toluene filtrate, which was then stirred for 2 hours at 90 °C. The HCl gas which evolved from the reaction was "trapped" in an alkali bath. The mixture was then cooled to room temperature and purged with nitrogen to remove all of the remaining HCl present in the solution.

At room temperature, a 5 ml aliquot of triethylamine was added to the concentrated toluene solution and left for a few minutes, after which 6 ml of 2 M H₂NMe (12 mmol) was added a few drops at a time. The suspension was filtered and washed with 20 ml of toluene. The toluene filtrate and the toluene wash fraction were combined. The combined toluene fractions were evaporated to dryness and 30 ml of methanol was added. The methanol solution was left overnight at -35 °C wherein a white (2-methoxyphenyl)(phenyl)PN(CH₃)P(2-methoxyphenyl)(phenyl) precipitate was formed in the solution. The precipitated ligand was then isolated.

The precipitated ligand consisted of two isomers, a racemic isomer (the RR and/or the SS enantiomers of the ligand) and a meso isomer (the RS enantiomer of the ligand); the proportions of these two isomers were determined by ³¹P NMR with peaks at 63.18 and 64.8 ppm corresponding to the two different isomers respectively. The sample consisted of a mixture of both the racemic and the meso isomers having weight ratios of 92/8.

### Composition A'

(2-methoxyphenyl)(phenyl)PN(CH₃)P(2-methoxyphenyl)(phenyl) in a 1:1 molar ratio with CrCl₃(THF)₃ was prepared by stirring an equimolar mixture of CrCl₃(THF)₃ and ligand component A in toluene for 1 hour at 50°C, followed by evaporation of the solvent in vacuum and washing of the residue with pentane.

### Ligand component B (comparative)

The (2-methoxyphenyl)₂PN(CH₃)P(2-methoxyphenyl)₂ ligand was prepared by first forming a solution of 1.59 g (5 mmol) (2-methoxyphenyl)₂PNEt₂ in 20 ml diethyl ether. To this solution 10 ml of a 1 M HCl solution in diethyl ether (10 mmol HCl) was added under an inert atmosphere at room temperature. The suspension thus formed was stirred overnight. The diethyl ether was removed from the product under vacuum and 20 ml of dry toluene was added. The resulting solution was filtered and the toluene was removed from the filtrate under vacuum to yield a white solid (2-methoxyphenyl)₂PCl product.

A solution of 0.51 g (5 mmol) of triethylamine in 20 ml of dry dichloromethane was added to the (2-methoxyphenyl)₂PCl product. To the resulting mixture, 1.25 ml of a 2 M H₂NMe solution in THF (2.5 mmol) was added and allowed to stir overnight. The solvents were removed from the resulting solution in vacuo and 20 ml of dry toluene was added. The mixture was then filtered. The toluene was removed from the filtrate under vacuum, and 10 ml of methanol was added to the residue to produce a suspension, which was filtered once more, to leave the solid white (2-methoxyphenyl)₂PN(CH₃)P(2-methoxyphenyl)₂ product which was isolated.

### Composition B'

(2-methoxyphenyl)₂PN(CH₃)P(2-methoxyphenyl)₂ in a 1:1 molar ratio with CrCl₃(THF)₃ was prepared similarly to Composition A'.

### Ligand component C (comparative)

The (phenyl)₂PN(isopropyl)P(phenyl)₂ ligand was prepared by the following method. At 0 °C, under a nitrogen atmosphere, 15 ml triethylamine was added to 6.3 g (phenyl)₂PCl in 80 ml of dry dichloromethane. To the resulting mixture, 0.844 g isopropylamine was added and allowed to stir overnight at room temperature. The solvents were removed from the resulting solution in-vacuo and 50 ml of dry toluene was added. The mixture was then filtered over a small layer of silica. The toluene was removed from the filtrate under vacuum. The (phenyl)₂PN(isopropyl)P(phenyl)₂ product was isolated as a white solid. Crystallization from ethanol yielded (phenyl)₂PN(isopropyl)P(phenyl)₂ as white crystals.

### Ligand component D

The (phenyl)₂PN(isopropyl)P(2-methoxyphenyl)₂ ligand was prepared by the following method.

Under a nitrogen atmosphere, 12 ml triethylamine was added to 3.39 g isopropylamine in 10 ml dry toluene. To the resulting mixture, 5.15 ml (phenyl)₂PCl was slowly added and allowed to stir overnight at room temperature. The precipitate was removed by filtration. The solvents were removed from the resulting solution in vacuo. To the evaporation residue pentane was added and subsequently the solvent was removed in vacuo from the pentane solution, yielding (phenyl)₂PNH(isopropyl) as a colourless oil, which crystallized on standing at room temperature.

Under a nitrogen atmosphere, 3 ml triethyl amine was added to 0.9 g of the isolated (phenyl)₂PNH(isopropyl) in 5 ml of dry dichloromethane. To the resulting mixture, 1.1 g (2-methoxyphenyl)₂PCl was added and allowed to stir for a week at room temperature. To the resulting reaction mixture 5-10 ml of dry toluene was added. The precipitate was removed by centrifugation. The solvents were removed from the resulting solution in vacuo. The resulting mixture was first washed with pentane and thereupon stirred with methanol yielding a white solid. The white solid was washed with pentane and dried in vacuo. Yield 0.7 g of (phenyl)₂PN(isopropyl)P(2-methoxyphenyl)₂.

### Co-catalyst

The co-catalyst used in the experiments below was selected from:
methyl aluminoxane (MAO) in toluene, [Al] = 5.20%wt, supplied by Crompton GmbH, Bergkamen, Germany;
tetraisobutyl dialuminoxane (TIBAO) 30%wt in cyclohexane, [Al] = 5.44%wt, supplied by Witco Polymer Chemicals, Witco GmbH, Bergkamen, Germany.

### Examples 1 - 11

### Catalyst system preparation for simultaneous trimerization and tetramerization in a batch autoclave

In a Braun MB 200-G dry box the CrCl₃ 1:1 complexes of ligands A or B (i.e. Compositions A' or B', indicated in Table 1) were placed in a glass bottle. The catalyst precursor composition was converted into the catalyst solution by adding 3 or 1.5 mmol of MAO solution in toluene (ca. 1.6 g or 0.8 g MAO solution), followed by typically 4 g of dry toluene. Finally the bottle was sealed by a septum cap.

These catalyst solutions, or part of these solutions, were used in the simultaneous tri- and tetramerization reaction of ethylene.

Alternatively, chromium tris(acetylacetonate) (typically 30 µmol) and the amount of ligand component C or D, as indicated in Table 1, were placed in a glass bottle, to which dry toluene (typically 4 g) was added to obtain the catalyst precursor solution. Finally the bottle was sealed with a septum cap.

These catalyst precursor solutions, or part of these solutions, were introduced to the autoclave as catalyst precursor solutions and activated by the pre-dosed MAO or TIBAO in-situ and subsequently used in the simultaneous tri- and tetramerization reaction of ethylene.

### Simultaneous trimerization and tetramerization reactions of ethylene in a 1.0-litre batch autoclave

Simultaneous tri- and tetramerization experiments were performed in a 1.0-litre steel autoclave equipped with jacket cooling with a heating/cooling bath (ex. Julabo, model ATS-2) and a turbine/gas stirrer and baffles.

The reactor was scavenged by introducing 250 ml toluene, MAO (0.6 g solution) or a similar amount of a TIBAO-solution, and subsequent stirring at 70°C under nitrogen pressure of 0.4-0.5 MPa for 30 min. The reactor contents were discharged via a tap in the base of the autoclave. The reactor was evacuated to about 0.4 kPa and loaded with approximately 250 ml toluene, heated to 40 °C and pressurised with ethylene to 15 barg or as indicated in Table 1.

Whilst stirring, a MAO-solution (typically an intake of 3.12 g, 6 mmol Al) or a TIBAO-solution as indicated in Table 1, was added to the reactor with the aid of toluene to attain an Al/Cr atomic ratio of 200 (typically, the total volume injected was about 25 ml: the MAO-solution diluted with toluene to 8 ml was injected and the injector system was rinsed twice with about 8 ml toluene) and the stirring at 800 rpm was continued for 30 minutes.

The Cr-catalyst precursor system (typically 30 µmol on Cr intake), prepared as described above, was introduced into the stirred reactor using an injection system with the aid of toluene (the total volume injected was about 25 ml: the catalyst solution diluted with toluene to 8 ml was injected and the injector system was rinsed twice with about 8 ml toluene). The initial loading of the reactor was about 300 ml of largely toluene.

The addition of the catalyst system resulted, after an induction period of some 5 minutes, in an exotherm (generally some 5-10°C), which generally reached a maximum within 1 minute and was followed by establishment of the temperature of 40°C and the pressure of 15 barg, unless indicated differently in Table 1.

After consuming the desired volume of ethylene, the simultaneous tri- and tetramerization was stopped by rapid cooling to room temperature (in about 5 minutes), followed by venting of the ethylene, decanting the product mixture into a collection bottle using a tap in the base of the autoclave. Exposure of the mixture to air resulted in rapid deactivation of the catalyst.

After addition of *n*-hexylbenzene (0.5-3.5 g) as internal standard to the crude product, the amount of the C₄-C₃₀ olefins and purity of C₆, C₈ and C₁₀ olefins was determined by gas chromatography. The experimental data is reported in Table 1.

In the case of experiments under 30 barg of ethylene pressure a similarly equipped 0.5-litre steel autoclave has been used, loaded (similarly to the above-described procedure for the 1.0-litre autoclave) with 150 ml of toluene, a MAO-solution or a TIBAO-solution and a Cr-catalyst system. The amounts of the Cr-catalyst system, MAO-, TIBAO-solution, solvent and ethylene consumption were typically half of those used in the corresponding 1.0-litre experiments to maintain the same Al/Cr atomic ratio (of about 200) and final alpha olefin concentration as much as practicable.

The experimental data is provided in Table 1 below.

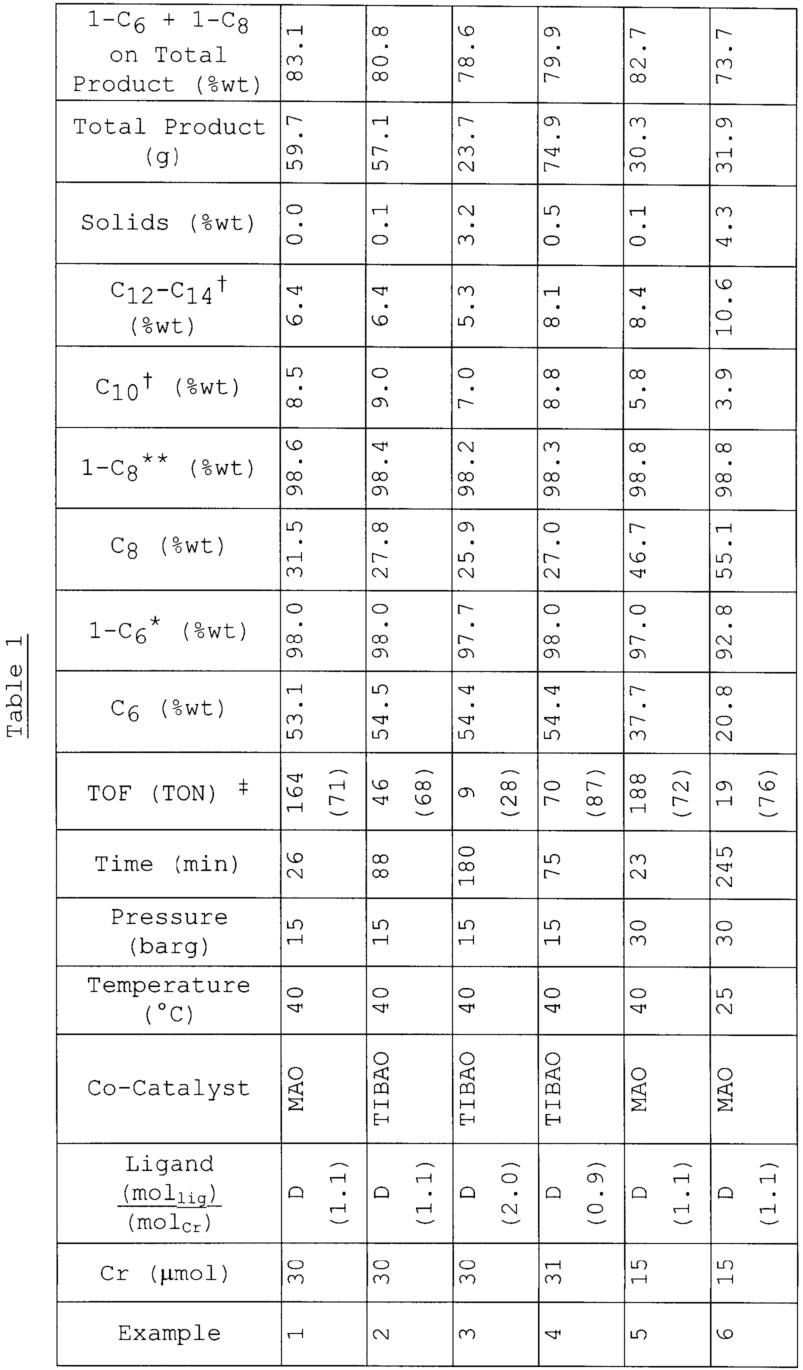

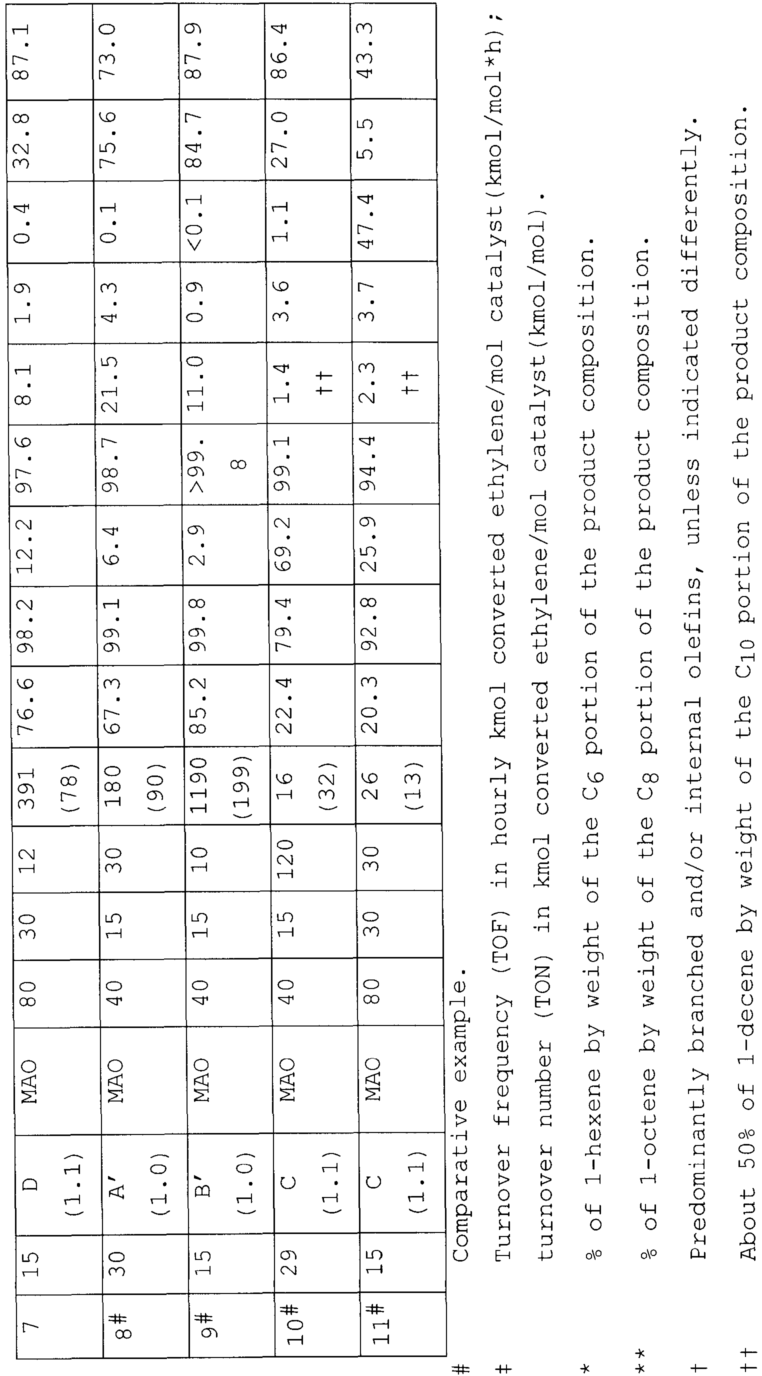

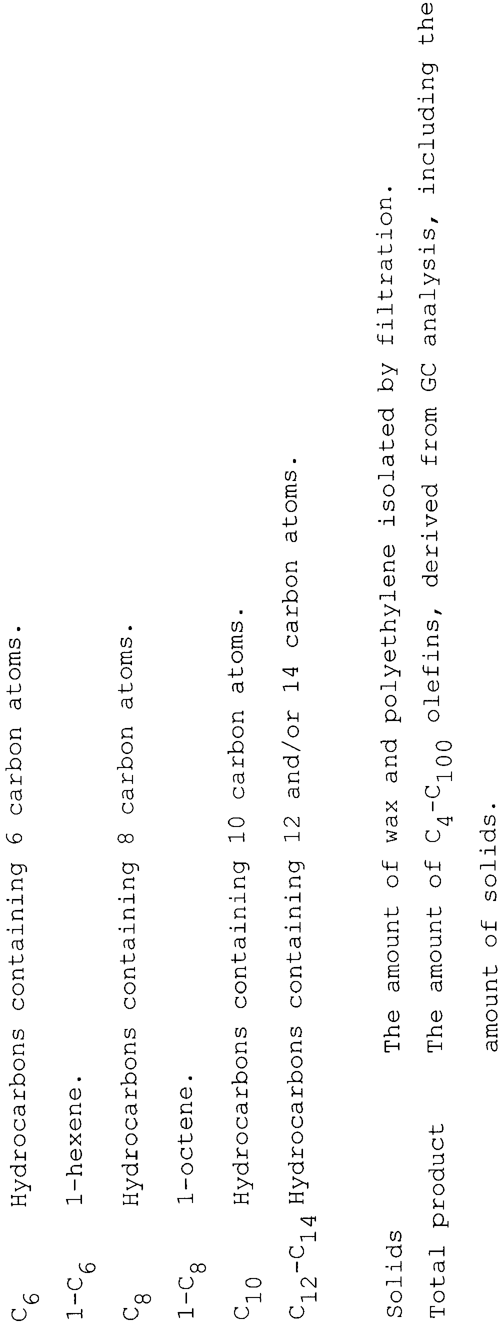

## Claims

1. A process for the simultaneous trimerization and tetramerization of olefinic monomers, wherein the process comprises contacting at least one olefinic monomer with a catalyst system comprising:
a) a source of chromium;
b) a ligand having the general formula (I);
(R¹)₂P-X-P(R¹)ₘ(R²)ₙ (I)
wherein:
X is a bridging group of the formula -N(R³)-, wherein R³ is selected from hydrogen, a hydrocarbyl group, a substituted hydrocarbyl group, a silyl group or derivative thereof;
the R¹ groups are independently selected from an optionally substituted aromatic group bearing a polar substituent on at least one of the ortho-positions; and
the R² groups are independently selected from substituted or unsubstituted aromatic groups, including substituted or unsubstituted heteroaromatic groups, which do not contain a polar substituent at any of the ortho-positions;
with the proviso that m is 0 or 1, n is 1 or 2 and the total of m + n is 2;
optionally, any of the R¹ and R² groups may independently be linked to one or more of each other or to the bridging group X to form a cyclic structure; and
c) a cocatalyst,
at a pressure in the range of from below atmospheric to 40 barg and at a temperature in the range of from 0 °C to 120 °C.

2. A process according to Claim 1, wherein R³ is selected from C₁-C₁₅ alkyl groups, substituted C₁- C₁₅ alkyl groups, C₂-C₁₅ alkenyl groups, substituted C₂-C₁₅ alkenyl groups, C₃-C₁₅ cycloalkyl groups, substituted C₃-C₁₅ cycloalkyl groups, C₅-C₁₅ aromatic groups and substituted C₅-C₁₅ aromatic groups, preferably C₁-C₁₅ alkyl groups.

3. A process according to any one of Claims 1 and 2, wherein m is 0 and n is 2.

4. A process according to any one of Claims 1 to 3, wherein the R2 groups are independently selected from optionally substituted phenyl groups which do not contain a polar substituent at any of the ortho-positions.

5. A process according to any one of Claims 1 to 4, wherein the R¹ group is independently selected from substituted or unsubstituted aromatic groups bearing an optionally branched C₁-C₂₀ alkoxy group on at least one of the ortho-positions, preferably an o-anisyl group.

6. A process according to any one of Claims 1 to 5, wherein the temperature is in the range of from 40 °C to 100 °C.

7. A process according to any one of Claims 1 to 6, wherein the amount of chromium, a), and the amount of ligand, b), are present in a molar ratio in the range of from 1:0.9 to 1:1.1.

8. A process according to any one of Claims 1 to 7, wherein the olefinic monomer is selected from ethylene, propylene, optionally branched C₄-C₂₄ α- olefins, optionally branched C₄-C₂₄ internal olefins, optionally branched C₄-C₂₄ vinylidene olefins, optionally branched C₄-C₂₄ cyclic olefins, optionally branched C₄-C₂₄ dienes, and optionally branched C₄-C₂₄ functionalized olefins.

9. A process according to Claim 8, wherein the olefinic monomer is ethylene.

## Patentansprüche

1. Verfahren zur gleichzeitigen Trimerisation und Tetramerisation von olefinischen Monomeren, wobei das Verfahren das Inkontaktbringen mindestens eines olefinischen Monomers mit einem Katalysatorsystem, das Folgendes umfasst:
a) eine Chromquelle;
b) einen Liganden mit der allgemeinen Formel (I):
(R¹) ₂P-X-P(R¹)ₘ(R²)ₙ (I),
wobei:
X eine Brückengruppe der Formel -N(R³)- ist, wobei R³ aus Wasserstoff, einer Hydrocarbylgruppe, einer substituierten Hydrocarbylgruppe, einer Silylgruppe oder einem Derivat davon ausgewählt ist;
die R¹-Gruppen unabhängig aus einer gegebenenfalls substituierten aromatischen Gruppe ausgewählt sind, die einen polaren Substituenten an mindestens einer der ortho-Stellungen trägt; und
die R²-Gruppen unabhängig aus substituierten oder unsubstituierten aromatischen Gruppen, einschließlich substituierten oder unsubstituierten heteroaromatischen Gruppen, ausgewählt sind, die keinen polaren Substituenten an einer beliebigen der ortho-Stellungen enthalten;
mit der Maßgabe, dass m 0 oder 1 ist, n 1 oder 2 ist und die Summe von m + n 2 ist;
gegebenenfalls eine beliebige der R¹- und R²-Gruppen unabhängig mit einer oder mehreren voneinander oder der Brückengruppe X verknüpft sein kann, um eine cyclische Struktur zu bilden; und
c) einen Cokatalysator,
bei einem Druck im Bereich von unterhalb Atmosphärendruck bis 40 bar ü und bei einer Temperatur im Bereich von 0 °C bis 120 °C umfasst.

2. Verfahren nach Anspruch 1, wobei R³ aus C₁-C₁₅-Alkylgruppen, substituierten C₁-C₁₅-Alkylgruppen, C₂-C₁₅-Alkenylgruppen, substituierten C₂-C₁₅-Alkenylgruppen, C₃-C₁₅-Cycloalkylgruppen, substituierten C₃-C₁₅-Cycloalkylgruppen, aromatischen C₅-C₁₅-Gruppen und substituierten aromatischen C₅-C₁₅-Gruppen, vorzugsweise C₁-C₁₅-Alkylgruppen ausgewählt ist.

3. Verfahren nach einem der Ansprüche 1 und 2, wobei m 0 ist und n 2 ist.

4. Verfahren nach einem der Ansprüche 1 bis 3, wobei die R²-Gruppen unabhängig aus gegebenenfalls substituierten Phenylgruppen ausgewählt sind, die keinen polaren Substituenten an einer beliebigen der ortho-Stellungen enthalten.

5. Verfahren nach einem der Ansprüche 1 bis 4, wobei die R¹-Gruppe unabhängig aus substituierten oder unsubstituierten aromatischen Gruppen ausgewählt ist, die eine gegebenenfalls verzweigte C₁-C₂₀-Alkoxygruppe an mindestens einer der ortho-Stellungen, vorzugsweise eine o-Anisylgruppe tragen.

6. Verfahren nach einem der Ansprüche 1 bis 5, wobei die Temperatur im Bereich von 40 °C bis 100 °C ist.

7. Verfahren nach einem der Ansprüche 1 bis 6, wobei die Menge an Chrom a) und die Menge an Ligand b) in einem Molverhältnis im Bereich von 1:0,9 bis 1:1,1 vorliegen.

8. Verfahren nach einem der Ansprüche 1 bis 7, wobei das olefinische Monomer aus Ethylen, Propylen, gegebenenfalls verzweigten C₄-C₂₄-α-Olefinen, gegebenenfalls verzweigten internen C₄-C₂₄-Olefinen, gegebenenfalls verzweigten C₄-C₂₄-Vinylidenolefinen, gegebenenfalls verzweigten cyclischen C₄-C₂₄-Olefinen, gegebenenfalls verzweigten C₄-C₂₄-Dienen und gegebenenfalls verzweigten funktionalisierten C₄-C₂₄-Olefinen ausgewählt ist.

9. Verfahren nach Anspruch 8, wobei das olefinische Monomer Ethylen ist.

## Revendications

1. Procédé de trimérisation et de tétramérisation simultanées de monomères oléfiniques, dans lequel le procédé comprend la mise en contact d'au moins un monomère oléfinique avec un système de catalyseur comprenant :
a) une source de chrome ;
b) un ligand ayant la formule générale (I) :
(R¹)₂P-X-P(R¹)ₘ(R²)ₙ (I)
dans laquelle :
X est un groupe pontant de formule -N(R³)-, dans laquelle R³ est choisi parmi un atome d'hydrogène, un groupe hydrocarbyle, un groupe hydrocarbyle substitué, un groupe silyle ou un dérivé de ceux-ci ;
les groupes R¹ sont choisis indépendamment parmi un groupe aromatique éventuellement substitué portant un substituant polaire sur l'une au moins des positions ortho ; et
les groupes R² sont choisis indépendamment parmi des groupes aromatiques substitués ou non substitués, incluant les groupes hétéroaromatiques substitués ou non substitués, qui ne contiennent pas de substituant polaire sur aucune des positions ortho ;
à condition que m valle 0 ou 1, n valle 1 ou 2 et le total de m + n valle 2 ;
éventuellement, l'un quelconque des groupes R¹ et R² peut être indépendamment lié à un ou plusieurs autres de ces groupes ou au groupe pontant X pour former une structure cyclique ; et
c) un co-catalyseur,
sous une pression dans la plage de inférieure à la pression atmosphérique à 40 barg et à une température dans la plage de 0°C à 120°C.

2. Procédé selon la revendication 1, dans lequel R³ est choisi parmi les groupes alkyle en C₁-C₁₅, les groupes alkyle en C₁-C₁₅ substitués, les groupes alcényle en C₂-C₁₅, les groupes alcényle en C₂-C₁₅ substitués, les groupes cycloalkyle en C₃-C₁₅, les groupes cycloalkyle en C₃-C₁₅ substitués, les groupes aromatiques en C₅-C₁₅ et les groupes aromatiques en C₅-C₁₅ substitués, de préférence les groupes alkyle en C₁-C₁₅.

3. Procédé selon l'une quelconque des revendications 1 et 2, dans lequel m vaut 0 et n vaut 2.

4. Procédé selon l'une quelconque des revendications 1 à 3, dans lequel les groupes R² sont choisis indépendamment parmi des groupes phényle éventuellement substitués qui ne contiennent pas de substituant polaire sur l'une quelconque des positions ortho.

5. Procédé selon l'une quelconque des revendications 1 à 4, dans lequel le groupe R¹ est choisi indépendamment parmi des groupes aromatiques substitués ou non substitués portant un groupe alcoxy en C₁-C₂₀ éventuellement ramifié sur l'une au moins des positions ortho, de préférence un groupe o-anisyle.

6. Procédé selon l'une quelconque des revendications 1 à 5, dans lequel la température est dans la plage de 40°C à 100°C.

7. Procédé selon l'une quelconque des revendications 1 à 6, dans lequel la quantité de chrome, a), et la quantité de ligand, b), sont présentes dans un rapport molaire dans la plage de 1:0,9 à 1:1,1.

8. Procédé selon l'une quelconque des revendications 1 à 7, dans lequel le monomère oléfinique est choisi parmi l'éthylène, le propylène, les α-oléfines en C₄-C₂₄ éventuellement ramifiées, les oléfines internes en C₄-C₂₄ éventuellement ramifiées, les vinylidène-oléfines en C₄-C₂₄ éventuellement ramifiées, les oléfines cycliques en C₄-C₂₄ éventuellement ramifiées, les diènes en C₄-C₂₄ éventuellement ramifiés et les oléfines fonctionnalisées en C₄-C₂₄ éventuellement ramifiées.

9. Procédé selon la revendication 8, dans lequel le monomère oléfinique est l'éthylène.
